(19) Europäisches Patentamt
European Patent Office
Office européen des brevets

(11) Veröffentlichungsnummer: **0 316 775 B1**

(12) **EUROPÄISCHE PATENTSCHRIFT**

(45) Veröffentlichungstag der Patentschrift: **20.05.92**

(51) Int. Cl.$^5$: **C08G 73/12**, C07D 207/452

(21) Anmeldenummer: **88118770.2**

(22) Anmeldetag: **10.11.88**

Verbunden mit 88910030.1/0389526
(europäische
Anmeldenummer/Veröffentlichungsnummer
durch Entscheidung vom 08.07.91.

# (54) Bismaleinimide, sowie daraus hergestellte Polyimide.

(30) Priorität: **18.11.87 AT 3039/87**

(43) Veröffentlichungstag der Anmeldung:
**24.05.89 Patentblatt 89/21**

(45) Bekanntmachung des Hinweises auf die Patenterteilung:
**20.05.92 Patentblatt 92/21**

(84) Benannte Vertragsstaaten:
**AT BE CH DE ES FR GB GR IT LI LU NL SE**

(56) Entgegenhaltungen:
**EP-A- 0 148 534**
**EP-A- 0 245 815**
**US-A- 3 380 964**
**US-A- 4 684 714**

(73) Patentinhaber: **PCD Polymere Gesellschaft m.b.H.**
**Danubiastrasse 21-25**
**A-2323 Schwechat-Mannswörth(AT)**

(72) Erfinder: **Horacek, Heinz**
**Bockgasse 43**
**A-4020 Linz(AT)**
Erfinder: **Greber, Gerd**
**Anzengruberstrasse 11**
**A-2540 Bad Vöslau(AT)**

(74) Vertreter: **Kunz, Ekkehard, Dr.**
**Chemie Holding AG Patentwesen St. Peter-Strasse 25**
**A-4021 Linz(AT)**

EP 0 316 775 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Polyimide auf Basis von Bismaleinimiden sind bereits aus der US-PS 3 380 964 bekannt, ihre Herstellung ist durch Erhitzen der Bismaleinimide bei Temperaturen von 80 bis 400 ° C möglich, wobei unter anderem Ethylen-bis-maleinimid, Diphenylmethan-bis-maleinimid, Diphenylether-bis-maleinimid oder Diphenylsulfon-bis-maleinimid eingesetzt werden.

Die bekannten Polyimide zeichnen sich durch eine für Kunststoffe sehr hohe Thermostabilität aus. Sie besitzen allerdings den Nachteil, daß sie sehr spröde und nur in begrenztem Umfang weiter verarbeitbar sind. Es lassen sich beispielsweise nur harte und spröde Laminate aus ihnen herstellen.

Die Aufgabe der Erfindung war es daher, Bismaleinimide zu finden, die zu flexiblen und weniger spröden Polyimiden polymerisiert werden können, die gegebenenfalls mit Fasern verstärkt sind, und gegebenenfalls durch anschließendes Formpressen in die endgültige Form gebracht werden können.

Die Lösung der Aufgabe wurde mit Hilfe von neuen Bismaleinimiden gefunden, die als Kettenelemente über -S- und -$SO_2$-Brücken verbundene Aromaten enthalten, und aus denen Polyimide mit verbesserten Eigenschaften hergestellt werden können.

Gegenstand der Erfindung sind demnach Bismaleinimide der Formel I worin R einen Rest der Formel II bedeutet.

Die Herstellung der erfindungsgemäßen Bismaleinimide erfolgt durch Umsetzung des Diamins der Formel III mit Maleinsäure oder einem Maleinsäurederivat. Bevorzugt setzt man das Diamin der Formel III mit Maleinsäureanhydrid um. Die Reaktion gelingt besonders gut in polaren Lösungsmitteln, wie z. B. Dimethylformamid, Dimethylacetamid, Dimethylsulfoxid, N-Methylpyrrolidon (NMP), Tetramethylharnstoff oder Hexamethylphosphorsäuretriamid.

Das Diamin der Formel III, 1,4-Bis-(4-aminophenylthio)diphenylsulfon (BDS), erhält man beispielsweise gemäß EP-A-0 245 815 durch Umsetzung des Na-Salzes von Bis-(4-mercaptophenyl)diphenylsulfon mit überschüssigem 4-Chlornitrobenzol zu 1,4-Bis-(4-nitrophenylthio)diphenylsulfon und anschließende Reduktion der Nitropgruppen.

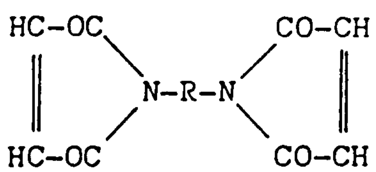

I

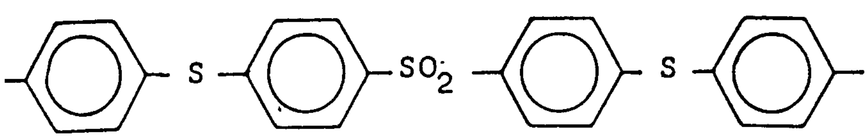

II

$H_2N$-R-$NH_2$ III

Die erfindungsgemäßen Bismaleinimide können zur Herstellung von Polyimiden mit verbesserten Eigenschaften verwendet werden. Die Bismaleinimide können entweder für sich allein, oder gemeinsam mit einem aromatischen Diamin zu Polyimiden polymerisiert werden. Als aromatische Diamine kommen alle in der Polyamid- und Polyimidchemie bekannten Diamine in Frage. Es eignen sich beispielsweise Diamine auf Basis von aromatischen, kondensierten aromatischen oder heteroaromatischen Ringen, die gegebenenfalls weiter substituiert sind, beispielsweise durch Alkyl- oder Alkoxygruppen oder durch Halogenatome. Das Diamin kann entweder aus einem oder aus mehreren Ringen aufgebaut sein, wobei die Ringe direkt oder über Brückenglieder miteinander verbunden sind. Geeignete Brückenglieder sind beispielsweise -O-, -$CH_2$-, -$C(CH_3)_2$, -$C(CF_3)_2$-, -S-, -SO-, -$SO_2$-, -CO-, -CO-O-, -CO-NH-, -N=N-, -NH-, -N(Alkyl)- mit 1 bis 6 C-Atomen im Alkylrest bzw. -N(Aryl)- mit 6 bis 20 C-Atomen im Arylrest.

Als aromatische Diamine kommen beispielsweise in Betracht:
1,4-Phenylendiamin, 4,4′-Diaminodiphenyl, 4,4′Diaminodiphenylmethan, 4,4′-Diaminodiphenylsulfon, 4,4′-Diaminodiphenylether, 4,4′-Diaminobenzophenon, 4,4′-Methylenbis(o-chloranilin), 1,2-Bis(2-aminophenylthio)propan, Methylendianthranilsäurediisopropylester, 4,4′-Methylen-bis-2,6-diisopropylanilin, 3,5-Diamino-4-chlorbenzoesäurepropylester, 4,4'-Diamino-5,5'-dimethylbiphenyl-2,2'-disulfonsäure, 5(6)-Amino-1-(4-aminophenyl)-1,3,3-trimethylindan, 5,5'-Diamino-2,2'-bipyridylsulfid, 2,4-Diethyltoluidindiamin, 4,4'-Diaminobisbenzophenon, Dioxyphenylenbisaminopyridin, 2,5-Diamino-1,3,4-thiadiazol.

Bevorzugt ist das Diamin der Formel III, das 1,4-Bis(4-aminophenylthio)diphenylsulfon.

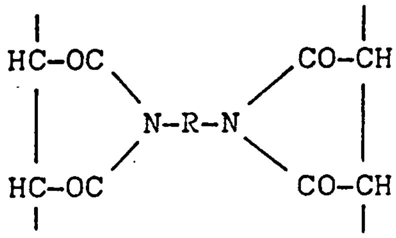

IV

Die aus den erfindungsgemäßen Bismaleinimiden hergestellten Polyimide sind ein weiterer Gegenstand der Erfindung. Die Polyimide bestehen aus sich wiederholenden monomeren Einheiten von Bismaleinimiden gemäß Anspruch 1 der Formel IV, wobei im Falle der Verwendung des Bismaleinimids allein die Polymerbindung über die Doppelbindungen der Maleinimidreste erfolgt, und im Falle der Verwendung von Bismaleinimid und Diamin außerdem eine Anlagerung der $NH_2$-Gruppen des Diamins an die Doppelbindung des Maleinimidrestes erfolgt. Bevorzugt sind Polyimide, die aus 0 bis 1 Molen Diamin je 1 Mol Bismaleinimid aufgebaut sind, wobei ein molares Verhältnis von Diamin zu Bismaleinimid von 0,1 : 1 bis 0,6 : 1 besonders bevorzugt ist. Die Viskosität der erfindungsgemäßen Polyimide liegt im Bereich von 100 bis 1000 mPas, gemessen in 50 Gew.%igen, frisch zubereiteten Lösungen in NMP bei 23° C (Brookfield Viscosimeter, Spindel 3).

Die erfindungsgemäßen Polyimide besitzen eine gute Temperaturbeständigkeit bis etwa 400° C, sowie sehr gute mechanische und elektrische Eigenschaften, so vor allem eine sehr hohe Schlagzähigkeit. Die mechanischen Eigenschaften zeigen bis zu Temperaturen von etwa 250° C keinen nennenswerten Abfall.

Aufgrund ihrer Löslichkeit, vor allem in den oben genannten polaren Lösungsmitteln, sowie aufgrund ihrer Schmelzbarkeit in nicht vollständig ausgehärtetem Zustand, können sie sehr einfach weiterverarbeitet werden, beispielsweise zu Fasern, Folien, Platten oder Preßteilen. Dabei können die Polyimide für bestimmte Anwendungen zur weiteren Verbesserung der mechanischen Eigenschaften vorteilhaft mit Fasern verstärkt werden, wobei Kurzfasern, Fasermatten, Gewebe oder unidirektionale Faserrovings bzw. Fasergelege zur Verstärkung eingesetzt werden können. Der Anteil an Verstärkungsfasern im Verbund liegt dabei üblicherweise bei etwa 20 - 70 Gew.%. Die Fasermatten können entweder aus Schnittfasern oder aus zu einem Vlies abgelegten Endlosfäden bestehen, wobei die Fasern und Fäden sowohl in Wirrlage als auch gerichtet vorliegen können. Die Verfestigung der Fasermatten erfolgt vorzugsweise mechanisch, beispielsweise durch Vernadeln, Vernähen oder Versteppen. Das Flächengewicht der Fasermatten liegt üblicherweise bei 250 -1.200 $g/m^2$, jenes von Geweben bevorzugt bei 50 -300 $g/m^2$.

Entsprechend den Ansprüchen an die mechanischen Eigenschaften können beispielsweise Glasfasern, Kohlefasern oder Aramidfasern, bzw. Mischungen aus diesen Fasern eingesetzt werden. Kohlefasern oder Aramidfasern kommen vor allem dort zum Einsatz, wo insbesondere an Festigkeit, Steifigkeit und geringes spezifisches Gewicht der aus den Polyimiden hergestellten Teile besondere Anforderungen gestellt werden.

In vielen Fällen erweist es sich als besonders vorteilhaft, die Polyimide nicht vollständig auszuhärten, sodaß sie noch weiter verarbeitbar sind, also noch verformbar, schmelzbar und hitzehärtbar bleiben, und gegebenenfalls in einem nachfolgenden Heißpreßvorgang zu einem Fertigteil umgeformt werden können. Dies ist im Falle von Prepregs von Bedeutung, die aus beispielsweise mit Fasergeweben oder Rovings verstärkten, nur teilweise ausgehärteten Polyimiden bestehen, lagerfähig sind und auch noch nach Monaten zum gewünschten Fertigteil heißverpreßt werden können.

Die Herstellung der erfindungsgemäßen Polyimide erfolgt durch Erhitzen des erfindungsgemäßen Bismaleinimids der Formel I, gegebenenfalls gemeinsam mit einem aromatischen Diamin auf Temperaturen von etwa 100 bis 300° C. Entsprechend der gewählten Temperatur und der Dauer des Erhitzens erfolgt eine mehr oder weniger vollständige Aushärtung des Polyimidharzes. Das Erhitzen erfolgt bevorzugt in zwei Stufen, wobei man in der ersten Stufe je nach Dauer des Erhitzens auf Temperaturen von etwa 100 bis 180° C erhitzt und dabei ein nicht vollständig ausgehärtetes Polyimid erhält. Man kann beispielsweise Bismaleinimid und Diamin 10 Minuten bei 140° C, bzw. 5 Minuten bei 150° C, bzw. 2 Minuten bei 165° C erhitzen, wobei die Ausgangsmaterialien versintern und teilweise polymerisieren. Die Ausgangsmaterialien werden vor und nach dem Versintern vorteilhafterweise gemahlen. Das nicht vollständig polymerisierte

Polyimid wird gegebenenfalls mit einer Faserverstärkung zusammengebracht, und kann anschließend in einer zweiten Stufe unter weiterem Erhitzen bei Temperaturen von 120 bis 300 °C und einem Druck von 1 bis 20 bar, vollständig ausgehärtet werden.

Eine vollständige Aushärtung kann beispielsweise bei einer Wärmebehandlung von 60 Minuten bei 200 °C, bzw. von 30 Minuten bei 250 °C und einem Druck von 15 bar erreicht werden. Zur Erzielung der optimalen mechanischen Eigenschaften ist eine anschließende Temperung während mehrerer Stunden notwendig. Es kann beispielsweise während 48 Stunden bei 200 °C, bzw. während 24 Stunden bei 250 °C, drucklos, getempert werden.

Zur Herstellung von faserverstärkten Polyimidharzen können beispielsweise die Verstärkungsfasern mit dem Bismaleinimid und gegebenenfalls dem Diamin zusammengebracht und anschließend unter Bildung des Polyimidharzes erhitzt werden. Es ist auch möglich, die Fasern mit dem bereits teilweise ausgehärteten Polyimidpulver zusammenzubringen. Die Herstellung faserverstärkter Laminate oder Prepregs erfolgt beispielsweise durch Aufbringen des fein gemahlenen Pulvers, z. B. aus einem Schüttkasten, auf eine Faser-Verstärkungsbahn, oder durch Imprägnieren der Verstärkungsbahn mit einer Lösung, und anschließendes Erhitzen der Bahnen, gegebenenfalls unter Druck. Besonders vorteilhaft gestaltet sich die kontinuierliche Herstellung von faserverstärkten Laminaten und Prepregs. Dabei dosiert man das pulverförmige, teilweise ausgehärtete Polyimid beispielsweise auf das Band einer Doppelbandpresse und läßt gleichzeitig eine oder mehrere Lagen der Faserverstärkung in Form eines Gewebes, eines Rovings, eines Geleges oder einer Matte mit in die Presse einlaufen. In der Doppelbandpresse erfolgt die Imprägnierung der Gewebe, Rovings, Gelege oder Matten mit dem aufgeschmolzenen Polyimid bei Drucken von 1 bis 20 bar und Temperaturen von 100 bis 300 °C, wobei je nach Verweilzeit und Temperatur auch eine vollständige Aushärtung möglich ist. Im Falle der Lösungsmittelimprägnierung wird die Verstärkungsbahn beispielsweise durch eine Wanne geführt, die eine etwa 40 - 60 Gew.%ige Lösung des teilweise ausgehärteten Polyimids in einem polaren Lösungsmittel, z. B. in N-Methylpyrrolidon, enthält. Die Auftragsmenge wird über Rakel und Abquetschwalzen eingestellt. Die Trocknung der imprägnierten Bahnen erfolgt beispielsweise in einem vertikalen oder waagrechten Trockentunnel. Die nur teilweise ausgehärteten faserverstärkten Polyimide (Prepregs) lassen sich in einem späteren Verarbeitungsschritt unter Druck- und Temperatureinwirkung zu einem vollständig ausgehärteten Fertigteil verarbeiten bzw. umformen, beispielsweise zu Leiterplatten, Elektrospulkernen, Teilen von Verbrennungsmotoren oder Geräteteilen für die Luft- und Raumfahrt.

Beispiel 1:

a) Herstellung des Bismaleinimids

In einem 250 ml Kolben wurden unter Stickstoffspülung 23,5 g (0,05 Mol) 1,4-Bis(4-aminophenylthio)-diphenylsulfon (BDS) in 80 g Dimethylformamid (DMF) gelöst und auf -20 °C abgekühlt. Daraufhin tropfte man unter Rühren 9,8 g (0,1 Mol) Maleinsäureanhydrid (MSA), gelöst in 20 g DMF, so langsam zu, daß die Temperatur des Reaktionsgemisches -15 °C nicht überschritt. Anschließend rührte man weitere 45 Minuten bei -15 °C, worauf man zu der orangefarbenen Lösung, die sich langsam braun färbte, bei -15 °C 1,0 g wasserfreies Na-acetat und 15 g Essigsäureanhydrid zufügte, die Lösung auf 55 °C erwärmte und 1 Stunde bei dieser Temperatur weiterrührte. Nach dem Abkühlen wurde die Lösung in ein Gemisch aus Eis und Wasser zugetropft, worauf ein brauner Niederschlag ausfiel, der abgesaugt, mit Wasser gewaschen und bei 80 °C im Vakuum getrocknet wurde.

Es wurden 28,4 g (90 % d. Th.) 1,4-Bis(4-maleinimidophenylthio)diphenylsulfonmit einem Fp von 140 °C erhalten.

b) Herstellung des Polyimids

1 Mol des gemäß Beispiel 1a erhaltenen Bismaleinimids wurde mit 0,4 Molen BDS vermahlen und anschließend während 10 Minuten bei 150 °C versintert. Das dabei erhaltene, schmelzbare Polyimid wurde zu einem Pulver gemahlen. Der Fp. lag bei 135 - 185 °C. Die Viskosität einer 50%igen frisch bereiteten Lösung in NMP betrug bei 23 °C 300 mPas (Brookfield Viskosimeter).

c) Herstellung eines Laminates

Auf ein 20 x 20 cm Glasgewebe (Nr. 92626, Fa. Interglas) mit einem Flächengewicht von 296 $g/m^2$ wurden 40 Gew.%, bezogen auf den fertigen Verbund, des in Beispiel 1 b erhaltenen Polyimdipulvers aufgebracht. Anschließend wurde 3 Minuten drucklos auf 200°C erhitzt, 1 Stunde bei 200°C und einem Druck von 15 bar verpreßt und schließlich 48 Stunden bei 200°C getempert.

Es wurde ein flexibles Laminat erhalten, die Eigenschaften sind in Tabelle 1 zusammengestellt.

Beispiel 2:

a) Herstellung des Polyimids

Das gemäß Beispiel 1a erhaltene Bismaleinimid wurde während 10 Minuten bei 200°C versintert. Das dabei erhaltene, schmelzbare Polyimid wurde zu einem Pulver vermahlen. Der Fp des gelben Pulvers lag bei 125 - 190°C.

b) Herstellung eines Laminates

Das gemäß Beispiels 2a erhaltene Polyimidpulver wurde analog zu Beispiel 1c in einer Menge von 40 Gew.% auf ein Glasgewebe aufgebracht, bei 200°C 1 Stunde bei 15 bar verpreßt und 48 Stunden getempert. Die Eigenschaften des erhaltenen Laminates sind in Tabelle 1 zusammengestellt.

Beispiel 3:

a) Herstellung des Polyimids

1 Mol des gemäß Beispiel 1a erhaltenen Bismaleinimids wurde mit 1 Mol BDS vermahlen und anschließend während 10 Minuten bei 150°C versintert. Das dabei erhaltene schmelzbare Polyimid wurde zu einem Pulver vermahlen. Der Fp lag bei 160 - 220°C.

b) Herstellung eines Prepregs

40 Gew.% des gemäß Beispiel 3a erhaltenen Polyimidpulvers wurden mit 60 Gew.% eines Glasgewebes (Nr. 92626, Fa. Interglas) bei 150°C und 15 bar Druck während 1 Minute zu einem thermoplastisch weiterverarbeitbaren Prepreg verpreßt.

c) Herstellung eines Laminates

Das gemäß Beispiel 3b erhaltene Prepreg wurde während 1 Stunde bei 200°C und 15 bar Druck verpreßt und anschließend während 48 Stunden bei 200°C drucklos getempert. Die Eigenschaften des erhaltenen Laminates sind in Tabelle 1 zusammengestellt.

Beispiel 4:

a) Herstellung des Polyimids

1 Mol des gemäß Beispiel 1a erhaltenen Bismaleinimids wurde mit 0,4 Molen 4,4′-Diaminodiphenyläther vermahlen und anschließend während 5 Minuten bei 150°C versintert. Das dabei erhaltene, schmelzbare Polyimid wurde anschließend zu Pulver vermahlen. Der Erweichungsbereich lag bei 160 - 300°C.

b) Herstellung eines Laminats

Auf ein 20 x 20 cm Glasgewebe (Nr. 92626, Fa. Interglas) wurden 40 Gew.%, bezogen auf den fertigen Verbund, des in Beispiel 4a erhaltenen Polyimidpulvers als 50 %ige Lösung in NMP aufgetragen. Anschließend wurde 3 Minuten drucklos auf 200°C erhitzt, 1 Stunde bei 200°C und einem Druck von 15 bar verpreßt und schließlich 48 Stunden bei 200°C getempert. Man erhält ein Laminat mit den physikalischen Werten der Tabelle 1.

Ein Laminat mit den gleichen Eigenschaften wurde erhalten, wenn das gemäß Beispiel 4a erhaltene Polyimid als Pulver auf das Glasfasergewebe aufgetragen wurde.

Tabelle 1

| | | Beispiel | | | |
|---|---|---|---|---|---|
| | | 1 | 2 | 3 | 4 |
| Bismaleinimid:Diamin | (Mol:Mol) | 1 : 0,4 | 1 : 0 | 1 : 1 | 1 : 0,4 |
| Diamin | | BDS[1] | BDS[1] | BDS[1] | DAE[2] |
| Zugfestigkeit DIN 53455 | $N/mm^2$ | 520 | 450 | 215 | 290 |
| Dehnung DIN 53455 | % | 5,2 | 2,1 | 7,0 | 1,5 |
| Biege-E-Modul DIN 53457 | $N/mm^2$ | 28000 | 32000 | 15000 | 23000 |
| Harzgehalt | % | 40 | 40 | 40 | 40 |
| Rohdichte | $g/cm^3$ | 1,69 | 1,72 | 1,70 | 1,71 |
| Schlagzugzähigkeit DIN 53453 n. Izod | J/m | 950 | 1000 | 1200 | 1100 |
| Druckfestigkeit DIN 53454 | $N/mm^2$ | 340 | 400 | 250 | 380 |
| Delaminierfestigkeit ASTM-D-2345 | $N/mm^2$ | 16 | 14 | 17 | 18 |
| Spez.Durchgangs-widerstand ASTM-D-257 1kHZ | Ohm.cm | $6.10^{14}$ | $5,5.10^{14}$ | $5.10^{14}$ | $6.10^{14}$ |
| Dielektrizitäts-konstanze ASTM-D-150 1kHZ | | 4,6 | 4,7 | 4,8 | 4,6 |
| Dielektr. Verlust-faktor tan delta bei 1kHZ | | $1,1.10^{-2}$ | $1,0.10^{-2}$ | $1,3.10^{-2}$ | $1,2.10^{-2}$ |

[1] 1,4-Bis(aminophenylthio)diphenylsulfon

[2] 4,4'-Diaminodiphenylether

**Patentansprüche**

1. Bismaleinimide der Formel I, worin R einen Rest der Formel II bedeutet.

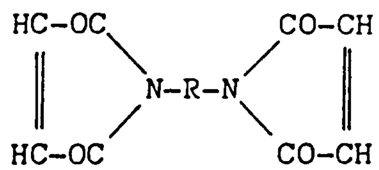

I

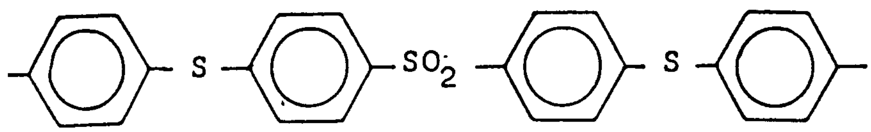

II

2. Verfahren zur Herstellung des Bismaleinimids gemäß Anspruch 1, dadurch gekennzeichnet, daß man ein Diamin der Formel III mit Maleinsäure oder einem Maleinsäurederivat umsetzt.

$H_2N\text{-}R\text{-}NH_2$ III

3. Verwendung von Bismaleinimiden gemäß Anspruch 1 zur Herstellung von Polyimiden, bestehend aus sich wiederholenden monomeren Einheiten von Bismaleinimiden der Formel IV, wobei an die Maleinimidgruppen zusätzlich 0 bis 1 Mol eines aromatischen Diamins pro 1 Mol Bismaleinimid angelagert sind.

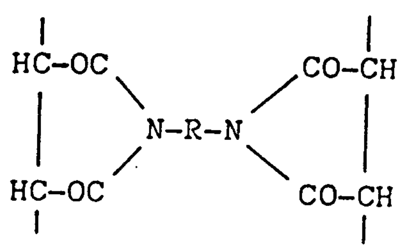

IV

4. Polyimide, bestehend aus sich wiederholenden monomeren Einheiten von Bismaleinimiden gemäß Anspruch 1 der Formel IV, worin an die Maleinimidgruppen zusätzlich pro 1 Mol Bismaleinimid 0 bis 1 Mole eines aromatischen Diamins angelagert sind.

5. Polyimide gemäß Anspruch 4, dadurch gekennzeichnet, daß sie pro 1 Mol Bismaleinimid 0,2 bis 0,6 Mole eines aromatischen Diamins enthalten.

6. Polyimide gemäß Anspruch 4 oder 5, dadurch gekennzeichnet, daß sie das aromatische Diamin gemäß Formel III enthalten.

7. Polyimide gemäß einem der Ansprüche 4 bis 6, dadurch gekennzeichnet, daß sie mit Fasern verstärkt sind.

**8.** Polyimide gemäß Anspruch 7, dadurch gekennzeichnet, daß sie in Form von Bahnen vorliegen und mit Geweben, Gelegen, Rovings oder Matten verstärkt sind.

**9.** Polyimide gemäß einem der Ansprüche 4 bis 8, dadurch gekennzeichnet, daß sie nicht vollständig ausgehärtet sind.

**10.** Verfahren zur Herstellung von Polyimiden gemäß einem der Ansprüche 4 bis 9, dadurch gekennzeichnet, daß man das Bismaleinimid der Formel I gemeinsam mit 0 bis 1 Mol eines aromatischen Diamins pro 1 Mol Bismaleinimid auf 100 bis 180 °C erhitzt, wobei man ein nicht vollständig ausgehärtetes Polyimid erhält, das gegebenenfalls mit einer Faserverstärkung zusammengebracht und anschließend gegebenenfalls bei einem Druck von 1 bis 20 bar auf eine Temperatur von 120 bis 300 °C erhitzt wird.

**Claims**

**1.** Bismaleimide of the formula I

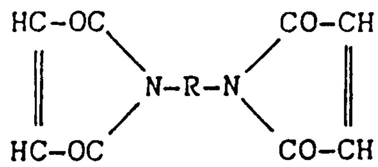

I

in which R denotes a radical of the formula

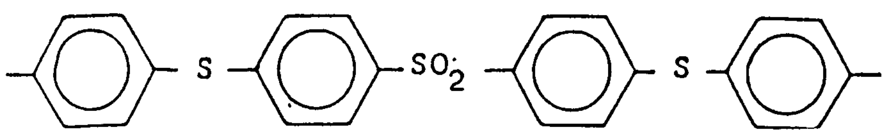

II

**2.** Process for the preparation of a bismaleimide according to Claim 1, characterised in that a diamine of the formula III

$H_2N$-R-$NH_2$ III

is reacted with maleic acid or a maleic acid derivative.

**3.** Use of a bismaleimide according to Claim 1 for the preparation of polyimides consisting of recurring monomeric units of a bismaleimide of the formula IV

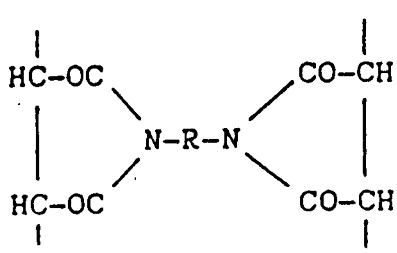

IV

0 to 1 mol of an aromatic diamine per mol of bismaleimide additionally being added onto the maleimide groups.

**4.** Polyimide consisting of recurring monomeric units of a bismaleimide according to Claim 1 of the formula IV

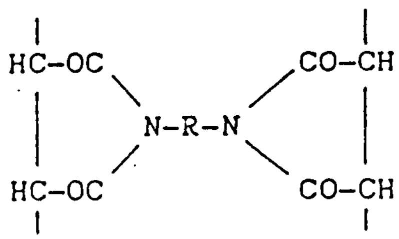

IV

in which 0 to 1 mol of an aromatic diamine is additionally added onto the maleimide groups per mol of bismaleimide.

**5.** Polyimide according to Claim 4, characterised in that it contains 0.2 to 0.6 mol of an aromatic diamine per mol of bismaleimide.

**6.** Polyimide according to Claim 4 or 5, characterised in that it contains the aromatic diamine of the formula III

$H_2N$-R-$NH_2$ III

**7.** Polyimide according to one of Claims 4 to 6, characterised in that it is reinforced with fibres.

**8.** Polyimide according to Claim 7, characterised in that it is present in the form of webs and is reinforced with fabrics, linings, roving or mats.

**9.** Polyimide according to one of Claims 4 to 8, characterised in that it is not fully hardened.

**10.** Process for the preparation of a polyimide according to one of Claims 4 to 9, characterised in that the bismaleimide of the formula I is heated together with 0 to 1 mol of an aromatic diamine per mol of bismaleimide, at 100 to 180 °C, an incompletely hardened polyimide being obtained which, if appropriate, is brought together with a fibre reinforcement and then heated, if appropriate, at a temperature of 120 to 300 °C under a pressure of 1 to 20 bar.

**Revendications**

**1.** Bismaléinimide de formule (I) :

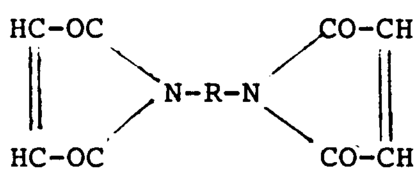

I

dans laquelle R signifie un reste de formule (II) :

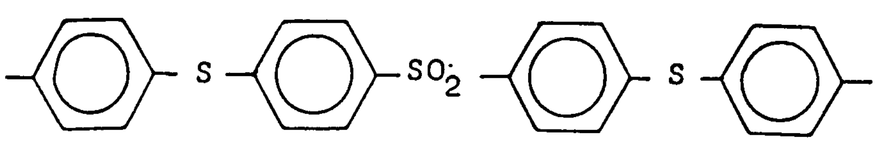

II

2. Procédé de préparation du bismaléinimide selon la revendications 1, caractérisé en ce qu'on fait réagir une diamine de formule (III) avec l'acide maléique ou bien un dérivé de l'acide maléique :

$H_2N\text{-}R\text{-}NH_2$ III

3. Utilisation du bismaléinimide selon la revendication 1 dans la préparation de polyimides, constitués d'unités monomères répétés de bismaléinimide de formule (IV) et additionnellement 0 à 1 mole de diamine aromatique par mole de bismaléinimide est liée aux groupes maléinimide.

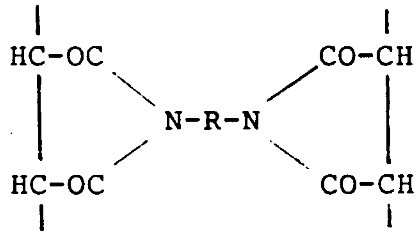

IV

4. Polyimides constitués d'unités monomères répétés de bismaléinimide selon la revendication 1, de formule (IV), où additionnellement 0 à 1 mole d'une diamine aromatique par mole de bismaléinimide est liée aux groupes maléinimide.

5. Polyimides selon la revendication 4, caractérisés en ce qu'ils renferment par mole de bismaléinimide 0,2 à 0,6 mole d'une diamine aromatique.

6. Polyimides selon la revendication 4 ou 5, caractérisés en ce qu'ils renferment la diamine aromatique de formule (III).

7. Polyimides selon l'une des revendications 4 à 6, caractérisés en ce qu'ils sont renforcés à l'aide de fibres.

8. Polyimides selon la revendication 7, caractérisés en ce qu'ils se présentent sous forme de lés, renforcés avec du tissu, une couche, du rovings ou du mat.

9. Polyimides selon l'une des revendications 4 à 8, caractérisés en ce qu'ils ne sont pas complètement durcis.

10. Procédé de préparation de polyimides selon l'une des revendications 4 à 9, caractérisés en ce qu'on chauffe le bismaléinimide de formule (I) avec 0 à 1 mole d'une diamine aromatique par mole de bismaléinimide de 100 à 180 ° C, pour obtenir alors un polyimide incomplètement durci, mélangé éventuellement avec un renforçateur de fibres et on chauffe ensuite le cas échéant à une pression de $10^5$ à $20.10^5$ Pa et à une température de 120 à 300 ° C.